Europäisches Patentamt

(19) European Patent Office

Office européen des brevets

(11) Publication number: **0 281 870**
**A2**

(12) # EUROPEAN PATENT APPLICATION

(21) Application number: **88102863.3**

(22) Date of filing: **26.02.88**

(51) Int. Cl.⁴: **A61L 2/20**

(30) Priority: **27.02.87 JP 44886/87**
**03.07.87 JP 166426/87**
**14.01.88 JP 6262/88**

(43) Date of publication of application:
**14.09.88 Bulletin 88/37**

(84) Designated Contracting States:
**AT BE CH LI LU NL SE**

(71) Applicant: **Masuda, Senichi, Prof.**
**3-2-1-415, Nishigahara Kita-ku**
**Tokyo-to(JP)**

(72) Inventor: **Masuda, Senichi, Prof.**
**3-2-1-415, Nishigahara Kita-ku**
**Tokyo-to(JP)**

(74) Representative: **Patentanwälte RUFF, BEIER**
**und SCHÖNDORF**
**Neckarstrasse 50**
**D-7000 Stuttgart 1(DE)**

(54) **Method for sterilizing objects to be sterilized and sterilizing apparatus.**

(57) A method for sterilizing objects to be sterilized is disclosed, in which an object to be sterilized such as a hand piece is preliminarily brought into a wetted state, and then accommodated and air-tightly closed up within a sterilizing chamber communicated with an oxygen cylinder, oxygen fed from the oxygen cylinder is introduced into an ozonizer to produce ozonized oxygen gas having an ozone concentration of at least 10000 ppm, preferably 15000 ppm or higher, then the ozonized oxygen gas is made to pass through the sterilizing chamber after it has been heated up to perfectly replace air within the sterilizing chamber by the ozonized oxygen gas, also moisture adhered to the objects to be sterilized is partly evaporated, subsequently the communication between the sterilizing chamber and the oxygen source is interrupted, the ozonized oxygen gas is circulated through the sterilizing chamber and the ozonizer while it is heated up at the inlet of the sterilizing chamber to thereby enhance the ozone concentration up to at least 20000 ppm, preferably up to 30000 ppm or higher wihtout consuming oxygen, and meanwhile, bacteria adhered to the objects to be sterilized are sterilized surely in a short period of time by the ozone gas under an inflated and wetted condition of the bacteria.

FIG. 1

## METHOD FOR STERILIZING OBJECTS TO BE STERILIZED AND STERILIZING APPARATUS

BACKGROUND OF THE INVENTION:

Field of the Invention:

The present invention relates to a method and an apparatus for sterilizing bacteria adhered onto a surface of a hand piece to be used by a dentist for treating a patient and onto an inner surface of an air communication bore therein and bacteria adhered to other objects to be sterilized by means of ozone gas.

Description of the Prior Art:

Heretofore, in a sterilizing apparatus for such type of objects, generally sterilization has been carried out through high-temperature heating with steam under a high pressure by making use of an autoclave. However, since the hand piece for a dentist is composed of precise mechanical parts such as a chuck for mounting and dismounting a small drill, an air turbine for rotating this chuck at a high speed, a device for cooling the tip end of the drill and the like, there is a fear that the precision may be possibly lost by distortion occurring upon high-temperature heating. Consequently, disinfection and sterilization cannot be carried out sufficiently, and it is surmised that a hygienically serious problem may be caused.

Furthermore, a time of about 15 minutes for heating up an object up to a temperature that is necessary for sterilization (about 130°C), a time of about 20 minutes for sterilizing perfectly at that temperature and a time of about 10 minutes for cooling the object, that is, in total a time of at least about 45 minutes was necessitated, hence quick sterilization of a hand piece could not be achieved, and this was quite inconvenient.

Besides the above-mentioned sterilizing apparatus, among the sterilizing apparatuses for the above-described type of objects an apparatus employing ethylene oxide gas is also known, but in this case also, a sterilizing time of at least 50 minutes and, in addition, repeated pressurizing and depressurizing operations for that gas were necessitated, and moreover, there was a shortcoming that ethylene oxide gas that is poisonous and carcinogenic might remain on a hand piece.

Though it can be conceived to carry out sterilization by making use of high-concentration ozone gas having a strong sterilizing power and no residual poisonousness in order to avoid the above-mentioned problems, in many cases a hand piece just after treatment for a patient would have its surface wetted and coated with a water film, and so, even if the hand piece under such condition is placed in ozone gas within a sterilizing chamber, the ozone gas would not fully reach bacteria in the water film and perfect sterilization was difficult.

In addition, in the case of certain one of the bacteria which can be hardly sterilized if the bacteria adhered to the surface of the hand piece are placed under a condition at a temperature lower than a room temperature or held at a dried condition, especially in the case of hey bacillus (Bacillus subtilis), since in their spore a cell membrane forming an outer wall of protoplasm maintains a dense shell structure, ozone gas is intercepted by the shell and cannot penetrate through the shell and reach the protoplasm therein, and therefore, it becomes very difficult to sterilize the bacteria. Furthermore, although it is relatively easy to sterilize a surface of a hand piece because it can be easily exposed to ozone gas, since an inner surface of an air communicating bore in a hand piece can be hardly exposed to ozone gas, it is practically very difficult to sterilize the inner surface.

SUMMARY OF THE INVENTION:

It is therefore one object of the present invention to resolve the above-mentioned respective problems in the case of carrying out sterilization by high-temperature heating with an autoclave or by making use of ethylene oxide gas or ozone gas, and especially to most effectively sterilize even spores of bacteria adhered to a hand piece.

Another object of the present invention is to preclude the above-described difficulties encountered in the case of sterilizing a hand piece under a wetted condition just after treatment by means of ozone gas and the adverse effect upon human bodies in the case of employing high-concentration ozone gas.

Still another object of the present invention is to provide an apparatus which facilitate to carry out sterilization of a surface of a hand piece as well as an inner surface of an air communicating bore therein.

A further object of the present invention is to improve a gas producing efficiency of an ozonizer and to design it in a compact form.

According to one feature of the present invention, there is provided a method for sterilizing objects to be sterilized such as a hand piece for use by a dentist, which consists of the steps of accommodating and air-tightly closing up objects to be sterilized such as a hand piece or the like within a sterilizing chamber communicated with an oxygen source such as an oxygen cylinder after they have been preliminarily made to be a wetted condition, producing ozonized oxygen gas having an ozone concentration of 10000 ppm or higher, preferably of 15000 ppm or higher by introducing oxygen fed from that oxygen source to an ozonizer, then replacing air within the sterilizing chamber completely by ozonized oxygen gas and also partly evaporating moisture adhered to the above-mentioned objects to be sterilized such as a hand piece or the like by making the produced ozonized oxygen gas pass through the sterilizing chamber after it has been heated up, subsequently interrupting the communication between the sterilizing chamber and the oxygen source and circulating the produced ozonized oxgen gas through the sterilizing chamber and the ozonizer while the produced ozonized oxygen gas is being heated up at an inlet of the sterilizing chamber to thereby enhance the ozone concentration at least up to 20000 ppm, preferably up to 30000 ppm or higher without consuming oxygen, and sterilizing bacteria adhered to the objects to be sterilized such as a hand piece or the like under an inflated and wetted condition by the above-mentioned ozone gas during the circulating period.

According to another feature of the present invention, there is provided an apparatus for sterilizing objects to be sterilized such as a hand piece for use by a dentist, wherein an oxygen source is communicated with one side of a sterilizing chamber via a heater and an ozonizer, an exhaust valve leading to the atmosphere is communicated with the other side of the sterilizing chamber via an ozone killer for decomposing residual ozone, further the ozonizer is coupled to the sterilizing chamber so that circulation of ozonized oxygen gas through the heater, the ozonizer and a circulating pump can be made, and said sterilizing chamber, the ozone killer, an ozone monitor and a circulating pump are communicated with one another so that circulation of ozonized oxygen gas can be made therethrough.

According to still another feature of the present invention, in the above-featured sterilizing apparatus, a receiving seat into which a base portion of a hand piece can be air-tightly inserted is provided within the sterilizing chamber, a communicating bore adapted to communicate with the communicating bore in the hand piece is formed in the receiving seat, the communicating bore is connected to a switching exhaust valve, one outlet of the switching exhaust valve is communicated with the outer atmosphere through the ozone killer, the other outlet of the same switching exhaust valve is communicated with an inlet side of the ozonizer through a circulating pump, and thereby it is made possible to exhaust the ozonized oxygen gas introduced into the sterilizing chamber through the interior of the communicating bore in the hand piece to the outer atmosphere or to feed it to the inlet of the ozonizer for making it circulate therethrough.

In operation, a hand piece for dental use is placed within a sterilizing chamber after its surface is preliminarily kept in a wetted condition by wiping it with wet sanitary cotton or wet paper napkin, a door of the chamber is closed, oxygen fed from an oxygen source is passed through an ozonizer to produce ozonized oxygen gas having an ozone concentration of at least 10000 ppm, preferably 15000 ppm, after this produced ozonized oxygen gas has been heated up to a temperature range of 30° - 70°C, preferably a temperature in the proximity of 50°C, it is fed into the sterilizing chamber, then this gas is discharged to the outside via an ozone killer, and thereby air within the sterilizing chamber is completly replaced by the ozonized oxygen gas. During this period, a past of water films adhering onto the surface of the hand piece and onto the inner surface of the air communication bore therein evaporates, ozone is adsorbed onto the surface, resulting in formation of an ozone-water surface layer and rise of the temperature of the water film, and inflation and wetting of the shell of a cell membrane of bacteria existing in the water film would commence. Subsequently, while the above-mentioned ozonized oxygen gas is being heated up at the inlet of the sterilizing chamber, it is circulated through the ozonizer and the sterilizing chamber to enhance the ozone concentration of the ozonized oxygen gas within the sterilizing chamber up to at least 20000 ppm, preferably up to 30000 ppm or higher, then an ozone-water surface layer having a further high concentration is formed by means of the high-concentration ozone gas, thus as the drying process of the water film proceeds, this ozone-water surface layer comes into contact with all the bacteria which would become exposed on the surface of the hand piece and on the inner surface of the air communication bore therein, it enters the interior of the cell of the bacterium after penetrating through the outer shell in an inflated and wetted condition, and it surely sterilizes all the bacteria. After this sterilizing process has been finished, the ozonizer is stopped, the sterilizing chamber is communicated with the ozone killer, then the ozonized oxygen gas having finished sterilization in the sterilizing chamber is circulated through the ozone killer, an ozone sensor and the sterilizing chamber by any appropriate method such as driving a circulating pump or the like, during this period, in the ozone killer the ozone gas is removed by decomposition of the ozone gas by heating or by a catalyst, or by adsorption of the ozone gas by an adsorbent, an amount of residual ozone is detected by the ozone sensor, in the sterilizing chamber, the ozone gas having a high concentration is replaced by the ozone gas having a low concentration at the downstream of the ozone killer through a scavenging action, and the concentration is gradually lowered. After it has been confirmed by the ozone monitor that the concentration was so lowered that the ozone is harmless for human bodies, the door of the sterilizing chamber is opened, and the sterilized hand piece is taken out to be used again for treatment of a patient.

## BRIEF DESCRIPTION OF THE DRAWINGS:

Fig. 1 is a gas flow circuit diagram in a sterilizing apparatus for practicing the method of sterilization according to the present invention;

Fig. 2 is an enlarged longitudinal cross-section view of a part of the apparatus shown in Fig. 1;

Fig. 3 is a transverse cross-section view taken along line III-III in Fig. 2;

Fig. 4 is an enlarged cross-section view of a part of a hand piece in the apparatus shown in Fig. 1;

Figs. 5, 6 and 7, respectively, are enlarged cross-section views of a bacterium; and

Fig. 8 is a gas flow circuit diagram of another preferred embodiment of the sterilizing apparatus according to the present invention.

## DESCRIPTION OF THE PREFERRED EMBODIMENTS:

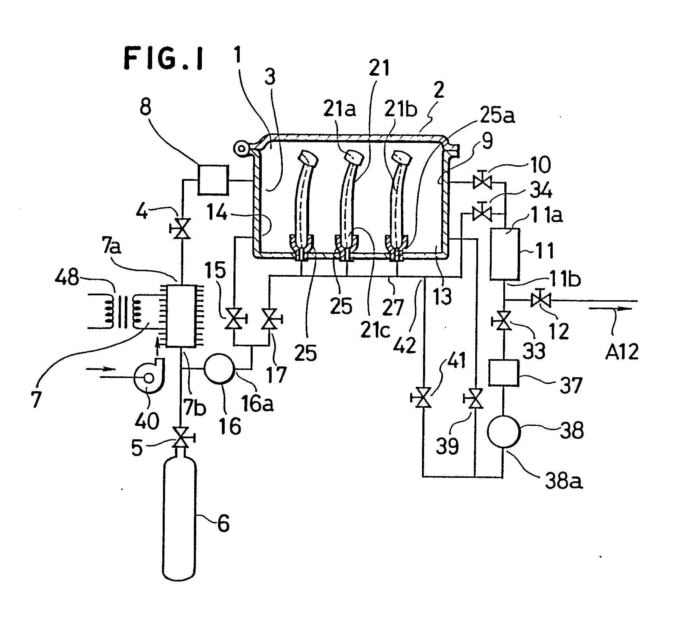

Figs. 1 to 3 illustrate a sterilizing apparatus for practicing the method for sterilization according to the present invention. With reference to these figures, in a sterilizing chamber 1, base portions 21c of hand pieces 21 held in a condition wetted by a water film adhering thereto after saliva, blood and the like of a patient have been wiped out with wetted sanitary cotton, wetted paper napkin, wetted paper towel or the like, are air-tightly fitted in receiving seats 25 to be accommodated in that chamber, and a lid 2 is sealingly closed. The receiving seat 25 has a communicating bore 25a and is communicated with a suction pipe 27, and this suction pipe 27 is connected to an inlet 7b of an ozonizer 7 via a valve 17 and a circulating pump 16 and connected to an inlet 11a of an ozone killer 11 via a valve 34. In addition, the suction pipe 27 is further connected to an outlet 11b of the ozone killer 11 via a valve 41, a circulating pump 38, an ozone monitor 37 and a valve 33.

Furthermore, the above-mentioned sterilizing chamber is provided with another gas inlet 13, which is connected to an outlet 38a of the circulating pump 38 via a valve 39, and the sterilizing chamber is further provided with still another gas outlet 14, which is connected to an inlet 16a of the circulating pump 16 via a valve 15.

Reference numeral 40 designates a fan for cooling the ozonizer 7.

At first, valves 4 and 5 are opened to make oxygen gas fed from an oxygen source 6 such as an oxygen cylinder pass through the ozonizer 7 to produce ozonized oxygen gas having an ozone concentration of at least 10000 ppm, thereafter the produced ozonized oxygen gas is heated up in a heater 8, the heated gas is fed into the sterilizing chamber 1 through a gas inlet 3 provided on one side of the sterilizing chamber 1 and passes through the sterilizing chamber 1, then it passes through a gas exhaust port 9 provided on the other side of the sterilizing chamber 1, and after it has passed through an exhaust valve 10 coupled to the gas exhaust port 9, the ozone killer 11 and a valve 12, oxygen gas having ozone removed therefrom is exhausted into the atmosphere in the direction of arrow A12. At this moment, valves 15, 17, 33, 34, 39 and 41 are held closed.

Thereby, air existed within the sterilizing chamber is replaced by ozonized oxygen gas.

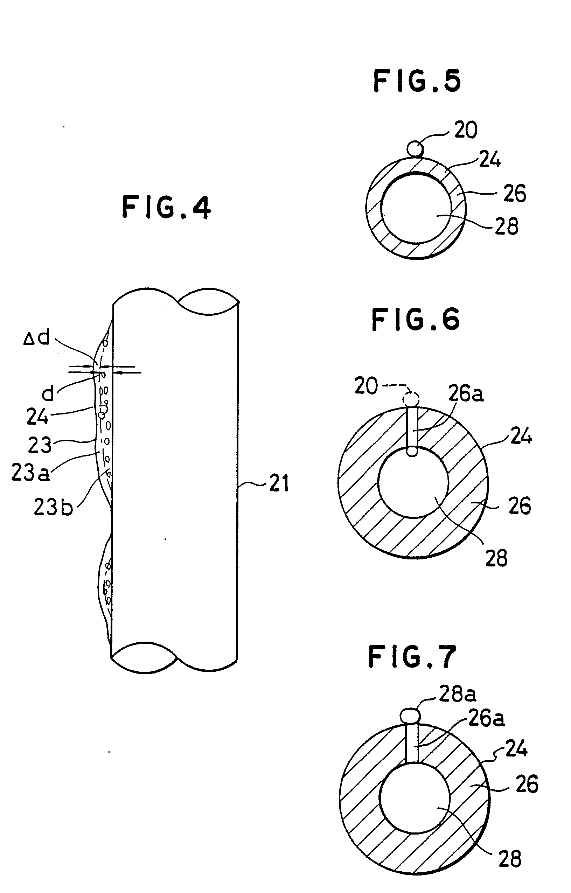

During this period, a water film 23 adhering onto the surface of the hand piece 21 is evaporated by the heated ozonized oxygen gas passing through the sterilizing chamber 1, and the thickness of the water film layer is reduced by $\Delta \underline{d}$ from the thickness of $\Delta \underline{d} + \underline{d}$ and thinned up to $\underline{d}$ as shown in Fig. 4.

Through this process, a surface layer 30 of the water film 23 absorbs ozone and a thin ozone-water layer 31 having a strong sterilizing power is formed here, but in its inner water layer 32, invasion of ozone does not occur, and since ozone-water is not produced therein, a sterilizing effect by ozone is not present there. Accordingly, at this step of the process, bacteria 24 in a most part of the inner water layer 32 except for the part of the surface layer 30 would not be subjected to a sterilizing effect.

However, the temperature of the water film 23 would rise as a result of contact with the heated ozonized oxygen gas, consequently the cell membrane of the bacterium is heated up under a wetted condition, resulting in inflation and wetting of the shell of the bacterium, and so, ozone in the ozone-water becomes easy to invade the interior as penetrating through the shell.

Next, when the valve 34 is opened and the valve 10 is closed, the heated ozonized oxygen gas introduced into the sterilizing chamber 1 enters through a head 21a of the hand piece 21 into an air communication bore 21 therein, then it passes the above-described communicating bore 25a, suction pipe 27, valve 34 and ozone killer 11 and is discharged to the outer atmosphere with the ozone content removed, and during this period, for the water film adhered to the inner surface of the communicating bore and bacteria in the water film, similarly to the above-described operation, partial evaporation of a water film, formation of a water surface layer, and inflation and wetting of a shell of the bacteria cell membrane are effected.

Subsequently, while repeating the operation of opening the valve 10 and closing the valve 34 and the operation of opening the valve 34 and closing the valve 10, replacement of air within the sterilizing chamber by ozonized oxygen gas is completed.

Next, by closing the valves 5, 17, 10 and 34 and opening the valve 15, the ozonized oxygen gas within the sterilizing chamber 1 is fed through the valve 15, the circulating pump 16, the ozonizer 7, the valve 4 and the heater 8 into the same chamber 1 from its gas inlet port 3, and thereby it is circulated.

Then, with the valve 17 opened and the valve 15 closed, the above-described ozonized oxygen gas is made to pass through the inner commuticating bore 21b of the hand piece 2, thereafter it is fed through the valve 17, the circulating pump 16, the ozonizer 7, the valve 4 and the heater 8 into the sterilizing chamber 1 via its gas inlet 3 and is thus circulated, and subsequently the operation of alternately opening and closing the valve 15 and valve 17, respectively, is repeated. In this way, the ozonized oxygen gas for sterilization use is circulated through the sterilizing chamber 1, the communicating bore 21b and the ozonizer 7, during this period the ozone concentration of the ozonized oxygen gas within the sterilizing chamber 1 is condensed up to at least 20000 ppm, preferably up to 30000 ppm or higher, this high-concentration ozone gas is brought into contact with the surface of water films adhered onto the outer surface of the above-described hand piece 21 and the inner surface of the communicating bore, and by greatly enhancing the ozone concentration of the ozone-water layer forming the surface layer the sterilizing power of the ozone-water layer can be raised greatly. Then, as the water film evaporates, bacteria 24 in the water film would be exposed gradually, and at this time the above-mentioned ozone-water layer would come into direct contact with the bacteria 24 being exposed. At this moment, under a wetted condition, the bacteria have been further inflated and wetted due to the temperature maintained by the ozone gas, and under such a condition, the high-concentration ozone would penetrate through the cell membrane extremely efficiently, and would enter the interior of the bacterium, and thereby it can completely sterilize the bacteria.

The sterilizing states within the sterilizing chamber at this moment are shown in Figs. 5, 6 and 7, among which Fig. 5 is an enlarged cross-section view of a bacterium 24 in a dried state, and by way of example, a spore of a hay bacillus (Bacillus subtilis) is illustrated. A cell membrane forming an outside portion of protoplasm 27 of the cell is constructed of a shell 26 which forms an extremely dense biological structure, hence even if an ozone molecule 20 should come into contact with its surface, it cannot reach the protoplasm 27 as intercepted by the shell 26, and so, it cannot sterilize.

In addition, Fig. 6 shows a state of the shell 26 that is wetted and inflated by applying heated ozone gas under a wetted condition. At this moment, an ozone molecule 20 on the outside of the shell 26 can quickly invade into the shell 26, the reaction speed for oxidizing the shell 26 is also very large, hence a penetration bore 26a is formed in a short period of time, thus the molecule 20 passes through the bore 26a and reaches the protoplasm 27, and it can immediately and perfectly sterilize.

Fig. 7 show a state of a sterilized bacterium 24, in which it can be confirmed with the aid of an electron microscope that a part 27a of the protoplasm 27 has been pushed out of the surface of the shell 26 through the above-described penetration bore 26a.

After the sterilization of the outer surface of the hand piece 21 as well as the inner surface of the communicating bore thereof has been finished in the above-described manner, the operations of the ozonizer 7, the circulating pump 16 and the heater 8 are stopped, the valves 4, 12, 15, 17 and 34 are closed, the valves 10, 33, 39 and 41 are opened, and the circulating pump 38 is operated. Then, the high-concentration ozonized oxygen gas in the sterilizing chamber 1 and within the communicating bore 21b of the hand piece 21 is circulated through the exhaust port 9, the valve 10, the ozone killer 11, the valve 33, the ozone monitor 37, the circulating pump 38, the valve 39 and the gas inlet 13, and also through the valve 41, the suction pipe 27 and the communicating bore 21b, and returns to the sterilizing chamber 1, and during this period an ozone concentration of the circulated gas is quickly lowered by the action of the ozone killer 11.

When this ozone concentration has been reduced to such extent that it becomes harmless for human bodies, this is confirmed by the ozone monitor 37, and then the lid 2 of the sterilizing chamber 1 is opened, and the hand pieces 21 accommodated therein are taken out for use.

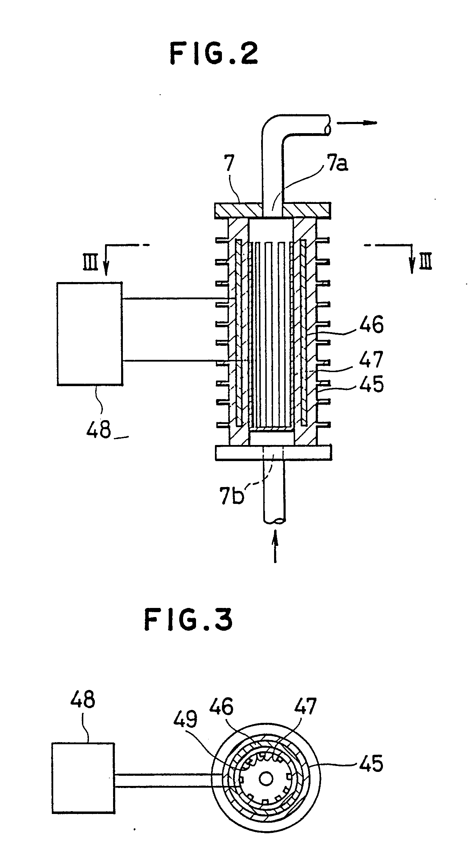

In the above-described ozonizer 7, as shown in Figs. 2 and 3, a planar dielectric electrode 46 is buried within a cylindrical dielectric body 45, wire-shaped corona discharge electrodes 47 are disposed on the inner surface of the cylindrical dielectric body 45, and a high-frequency high-voltage power supply 48 is connected between these respective electrodes 46 and 47. In operation, a high-frequency high voltage is applied between these electrodes 46 and 47, thereby creeping discharge 49 is generated on the surface of the cylindrical dielectric body 45 on the side of the wire-shaped corona discharge electrode 47, and the oxygen gas fed through its inlet 7b is transformed into ozone gas.

At this time, by cooling the ozonizer 7 by means of a cooling fan 40 as shown in Fig. 1, an ozone generating efficiency of the ozonizer 7 can be improved.

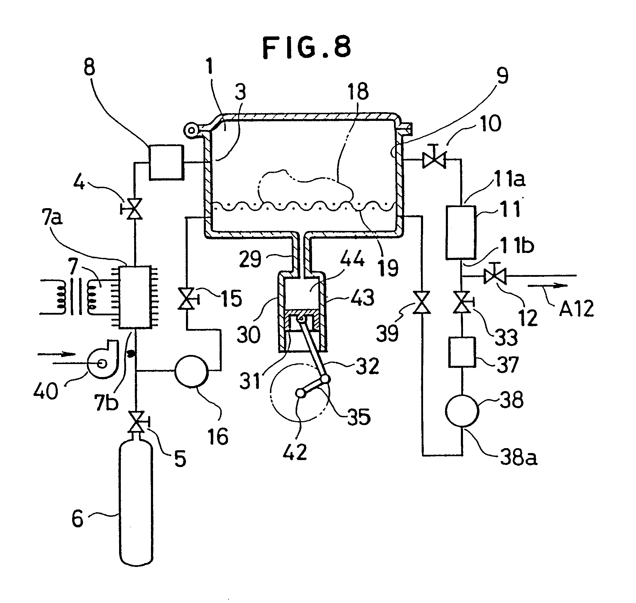

A sterilizing apparatus shown in Fig. 8 forms another preferred embodiment of the present invention, in which a sterilizing chamber 1 and a pressure chamber 44 of a pressure-vibrating device 43 are communicated with each other through a communicating pipe 29. In the present-vibrating device 43, a piston 31 is slidably fitted in a cylinder 30, the piston 31 and a crank shaft 42 are coupled via a connecting rod 32 and a crank arm 35, this crank shaft 42 is rotated by an electric motor or the like, and thereby pressure variation in the pressure chamber 44 caused by reciprocating the piston 31 within the cylinder 30 is transmitted into the sterilizing chamber 1 through the communicating pipe 29, as a vibrating force.

Thereupon, if all the valves 4, 10,39 and 15 communicating with the sterilizing chamber 1 are respectively closed with ozonized oxygen gas filled in the sterilizing chamber 1, then the pressure of the ozonized oxygen gas within the sterilizing chamber 1 would be vibrated by the above-mentioned vibrating force. Consequently, an object 18 to be sterilized on a wire net 19 that is preliminarily closed tightly in the sterilizing chamber 1 would be repeatedly subjected to pressurization and depressurization, hence the ozonized oxygen gas would come in and out deeply to and from the interior and even the interstices of the object to be sterilized, and it can perfectly sterilize the bacteria existing there.

In more particular, the above-described pressure vibrating device 43 should not be limited to the above-described construction consisting of a cylinder 30 and a piston 31, but in place of such construction, a combination of a conventional diaphragm and contracting/expanding means therefor, for instance, the assembly of the crank shaft 42, crank arm 35, connecting rod 32 and electric motor, with the cylinder 30 replaced by guide rails for the piston 31, could be employed. At this instance, an opening portion of that diaphragm is mounted to the communicating pipe 29 and the other end thereof is mounted to a slide portion corresponding to the piston 31.

It is to be noted that among the reference numerals used in the embodiment shown in Fig. 8, the same numerals as those used in Fig. 1 designate component parts having the same names and the same functions, and so, further explanation thereof has been omitted. But it will be obvious from the above description with reference to Fig. 1.

The present invention is as described above, and since an oxygen source and an ozonizer are coupled by piping with a sterilizing chamber via a heater, dried heated ozonized oxygen gas can be fed into the sterilizing chamber, hence if a hand piece held in a wetted state is brought into the sterilizing chamber, during the replacing process of the initial air in the sterilizing chamber by the ozonized oxygen gas, an ozone-water layer having a strong sterilizing power is initially formed in the surface portion of a water layer adhered onto the surface of the hand piece as well as onto the inner surface of the communicating bore thereof, thereby a sterilizable condition is started, and also a cell membrane or an outer shell on the surface of a bacterium can be brought into a wetted and inflated state where the bacterium can be easily sterilized.

During this process, since the ozonized oxygen gas produced in the ozonizer is circulated through the interior of the sterilizing chamber, the interior of the communicating bore in the hand piece and the ozonizer while being heated up especially in the next step of the process, the adhered water film is evaporated while further enhancing the ozone concentration of the above-mentioned ozone-water surface layer, the inflation and wetting of the outer shell of the bacterium is further promoted, and in the process of exposing the bacteria in accordance with evaporation of the water film, the ozone-water layer having the above-mentioned strong sterilizing power is made to act upon all the bacteria and can sterilize them perfectly. In other words, the high-concentration ozone molecules in the ozone-water surface layer can easily invade the outer shell of the bacterium that has been inflated and wetted under a wetted condition, thus forms a penetration bore in the outer shell by an oxidation reaction, hence the ozone can easily enter up to protoplasm of the bacterium, and thereby the bacteria can be sterilized in a short period of time.

In addition, according to the present invention, since the sterilizing chamber, the hand piece communicating bore and the ozone killer are so connected by piping that after completion of a sterilizing operation among them, the ozonized oxygen gas still containing high-concentration residual ozone can be circulated, by repeating this circulation, each time ozone that is harmful for human bodies is decomposed into oxygen in the ozone killer, and therefore, even if thereafter one opens the lid of the sterilizing chamber and takes out the hand piece in the chamber, there is no danger at all for human bodies and the apparatus is safe, because ozone gas would not be exhausted from the interior of the sterilizing chamber to the environmental atmosphere. Furthermore, since it is possible to open the lid and take out sterilized instruments after it has been confirmed by an ozone monitor that the ozone gas concentration has been sufficiently lowered, by coupling the ozone monitor with the ozone killer, there is no fear that ozone gas may flow out from the sterilizing chamber into the environmental atmosphere and may hurt human bodies when the lid is opened.

## Claims

1. A method for sterilizing objects to be sterilized, characterized by the steps of accommodating and air-tightly closing up objects to be sterilized in a wetted state within a sterilizing chamber, feeding high-concentration ozonized oxygen gas produced in an ozonizer coupled to said sterilizing chamber after it has been heated up and making it pass through said sterilizing chamber to partly evaporate moisture adhered to the surface of said objects to be sterilized while replacing the contents of said sterilizing chamber by said high-concentration ozonized oxygen gas, thereafter making said high-concentration ozonized oxygen gas circulate through said sterilizing chamber and said ozonizer, heating said high-concentration ozonized oxygen gas at the inlet of said sterilizing chamber when it is circulating to thereby further enhance its ozone concentration, evaporating moisture adhered to the surface of said objects to be sterilized while it is being heated, meanwhile forming a high-concentration ozone-water layer on the surface of a water layer adhered to the surface of said objects to be sterilized as a result of contact with said high-concentration ozonized oxygen gas, at the same time bringing a cell membrane of a bacterium adhering onto the surface of said objects to be sterilized into an inflated and wetted condition during said heating step in a wetted state, and during said adhered moisture evaporating step, making said high-concentration ozone-water layer come into contact with and act upon all the inflated and wetted bacteria which are being exposed during this step to perfectly sterilize the bacteria under an inflated and wetted condition.

2. A method for sterilizing a hand piece for dental use, characterized by the steps of accommodating and air-tightly closing up a hand piece in a wetted state within a sterilizing chamber, feeding high-concentration ozonized oxygen gas produced in an ozonizer coupled to said sterilizing chamber after it has been heated up and making it pass through said sterilizing chamber to partly evaporate moisture adhered to the surface of said hand piece while replacing the contents of said sterilizing chamber by said high-concentration ozonized oxygen gas, thereafter making said high-concentration ozonized oxygen gas circulate through said sterilizing chamber and said ozonizer, heating said high-concentration ozonized oxygen gas at the inlet of said sterilizing chamber when it is circulating to thereby further enhance its ozone concentration, evaporating moisture adhered to the surface of said hand piece while it is being heated, meanwhile forming a high-concentration ozone-water layer on the surface of a water layer adhered to the surface of said hand piece as a result of content with said high-concentration ozonized oxygen gas, at the same time bringing a cell membrane of a bacterium adhering onto the surface of said hand piece into an inflated and wetted condition during said heating step in a wetted state, and during said adhered moisture evaporating step, making said high-concentration ozone-water layer come into contact with and act upon all said inflated and wetted bacteria which are being exposed during this step to perfectly sterilize the bacteria under an inflated and wetted condition.

3. A method for sterilizing a hand piece for dental use, characterized by the steps of accommodating and air-tightly closing up a hand piece in a wetted state within a sterilizing chamber, feeding high-concentration ozonized oxygen gas produced in a ozonizer coupled to said sterilizing chamber after it has been heated up and making it pass through said sterilizing chamber and along an inner surface of a communicating bore in said hand piece to partly evaporate moisture adhered to the surface of said hand piece and to the inner surface of said communicating bore while replacing the contents therein by said high-concentration ozonized gas, thereafter making said high-concentration ozonized gas circulate through said sterilizing chamber and said ozonizer and also through the interior of the communicating bore in said hand piece and said ozonizer, heating said high-concentration ozonized oxygen gas at the inlet of said sterilizing chamber when it is circulating to thereby further enhance its ozone concentration, evaporating moisture adhered to the surface of said hand piece and to the inner surface of the communicating bore in said hand piece while it is being heated, meanwhile forming a high concentration ozone-water layer on the surface of the water layer adhering onto the surface of said hand piece and onto the inner surface of said communicating bore as a result of contact with said high-concentration ozonized oxygen gas, at the same time bringing a cell membrane of a bacterium adhering onto the surface of said hand piece and onto the inner surface of said communicating bore into an inflated and wetted condition during said heating step in a wetted state, and during said adhered moisture evaporating step, making said high-concentration ozone-water layer come into contact with and act upon all said inflated and wetted bacteria which are being exposed during this step to perfectly sterilize the bacteria under an inflated and wetted condition.

4. An apparatus for sterilizing objects to be sterilized, characterized in that an oxygen source is communicated with one side of a sterilizing chamber via a heater and an ozonizer, in exhaust valve leading to the atmosphere is communicated with the other side of said sterilizing chamber via an ozone killer, further said ozonizer is coupled to said sterilizing chamber so that circulation of ozonized oxygen gas through the heater, the ozonizer and a circulating pump can be made, and said sterilizing chamber, said ozone killer, an ozone monitor and a circulating pump are communicated with one another so that circulation of ozonized oxygen gas can be made therethrough.

5. An apparatus for sterilizing objects to be sterilized as claimed in Claim 4, characterized in that said sterilizing chamber is communicated with a pressure chamber of a pressure vibrating device.

6. An apparatus for sterilizing a hand piece for dental use, characterized in that in the sterilizing apparatus as claimed in Claim 4, a receiving seat into which a base portion of a hand piece can be air-tightly inserted, is provided within said sterilizing chamber, a communicating bore adapted to communicate with the communicating bore in said hand piece is formed in said receiving seat, said communicating bore is connected to a switching exhaust valve, one outlet of said switching exhaust valve is communicated with the outer atmosphere through the ozone killer, the other outlet of said switching exhaust valve is communicated with an inlet side of the ozonizer through a circulating pump, and thereby it is made possible to exhaust the ozonized oxygen gas introduced into the sterilizing chamber through the interior of said communicating bore in the hand piece to the outer atmosphere or to feed it to the inlet of the ozonizer for making it circulate therethrough.

FIG.1

0 281 870

# FIG.2

# FIG.3

## FIG.5

20
24
26
28

## FIG.4

Δd
d
24
23
23a
23b
21

## FIG.6

20
26a
24
26
28

## FIG.7

28a
26a
24
26
28

FIG.8